# EUROPÄISCHE PATENTSCHRIFT

(11) **EP 1 014 989 B1**
(45) Veröffentlichungstag und Bekanntmachung des Hinweises auf die Patenterteilung: **14.01.2004**
(21) Anmeldenummer: 98948954.7
(22) Anmeldetag: 10.09.1998
(51) Int. Cl.: A61K 31/505, A61P 9/00, A61P 9/08

(54) **ENDOTHELIN ANTAGONIST UND RENIN-ANGIOTENSIN SYSTEM HEMMER ALS KOMBINATIONSPRÄPARATE**
ENDOTHELIN ANTAGONIST AND A RENIN-ANGIOTENSIN SYSTEM INHIBITOR AS A COMBINED PREPARATION
ANTAGONISTE D'ENDOTHELINE ET SYSTEME RENINE-ANGIOTENSINE INHIBITEUR PRESENTES SOUS FORME DE PREPARATION PHARMACEUTIQUE

(30) Priorität: 26.09.1997 DE 19742717; 30.09.1997 DE 19743141
(43) Veröffentlichungstag der Anmeldung: 05.07.2000
(73) Patentinhaber: Abbott GmbH & Co. KG, 65205 Wiesbaden (DE)
(72) Erfinder: MÜNTER, Klaus, D-68163 Mannheim (DE); KIRCHENGAST, Michael, D-68161 Mannheim (DE); KORIOTH, Horst, D-69245 Bammental (DE)
(74) Vertreter: Grünecker, Kinkeldey, Stockmair & Schwanhäusser Anwaltssozietät
(86) Internationale Anmeldenummer: PCT/EP1998/005773
(87) Internationale Veröffentlichungsnummer: WO 1999/016445

(56) Entgegenhaltungen:
- EP-A- 0 617 001
- WO-A-96/19233
- WO-A-96/22978
- WO-A-98/09953
- WO-A-98/24482
- WO-A-98/27070
- US-A- 5 696 116
- CAMERON N. E. ET AL: "Effects of a nonpeptide endothelin-1 ETa antagonist on neurovascular function in diabetic rats: interaction with the renin-angiotensin system" J PHARMACOL EXP THER, Bd. 278, Nr. 3, 1996, Seiten 1262-1268, XP002093102
- BRUNNER H. R.: "Endothelin inhibition as a biological target for treating hypertension" AMERICAN JOURNAL OF HYPERTENSION, Bd. 11, Nr. 4, April 1998, Seiten 103s-109s, XP002093103

## Beschreibung

Die vorliegende Erfindung betrifft neue pharmazeutische Kombinationspräparate, die sich zur Behandlung von Herz-Kreislauf-Erkrankungen eignen und einen Hemmer des Renin-Angiotensin-Systems (RAS-Hemmer) und einen Endothelin-Antagonisten enthalten.

Kombinationspräparate, die sich zur Behandlung von Herz-Kreislauf-Erkrankungen eignen und die einen Hemmer des Renin-Angiotensin-Systems (RAS-Hemmer) und einen Endothelin-Antagonisten enthalten, sind bereits bekannt (EP-A-634 175, EP-A-617 001). Diese Wirkstoffmischungen sind jedoch in ihrer Wirkung unbefriedigend. Weitere Kombinationspräparate aus einem RAS-Hemmer und einem Endothelin-Antagonisten werden in der WO-A-96/22978 und der WO-A-98/09953 offenbart.

Aufgabe der vorliegenden Erfindung ist es daher, Wirkstoffmischungen mit verbesserten Eigenschaften zur Verfügung zu stellen.

Die Aufgabe wird gelöst durch die Kombination aus einem Endothelin-Antagonisten der Formel (I) 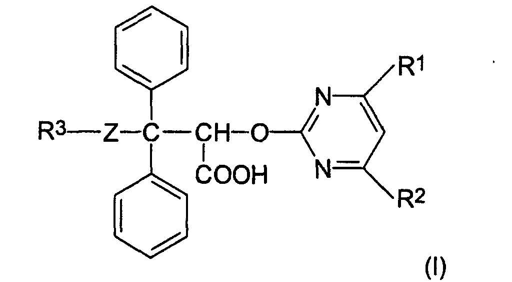 in der die Substituenten folgende Bedeutung haben:
- R¹: C₁-C₄-Alkyl, C₁-C₄-Alkoxy;
- R²: C₁-C₄-Alkyl, C₁-C₄-Alkoxy;
- R³: C₁-C₈-Alkyl;
- Z: Sauerstoff oder eine Einfachbindung;
und einem RAS-Hemmer.

Bevorzugt sind als Endothelin-Antagonisten diejenigen Verbindungen der Formel I, worin die Substituenten folgende Bedeutung haben:
- R¹:: C₁₋₂-Alkyl, C₁₋₂-Alkoxy
- R²:: C₁₋₂-Alkyl, C₁₋₂-Alkoxy
- R³:: C₁₋₂-Alkyl,
- Z:: Sauerstoff oder eine Einfachbindung.

Als Endothelin-Antagonisten eignen sich insbesondere folgende Verbindungen:

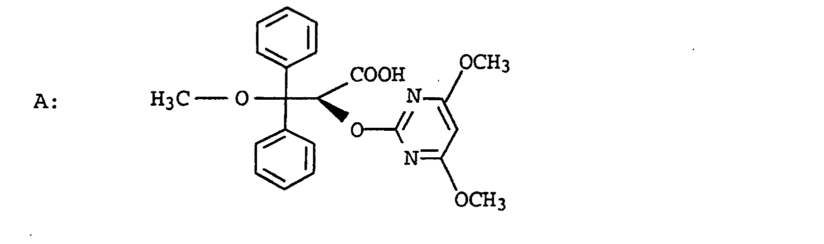

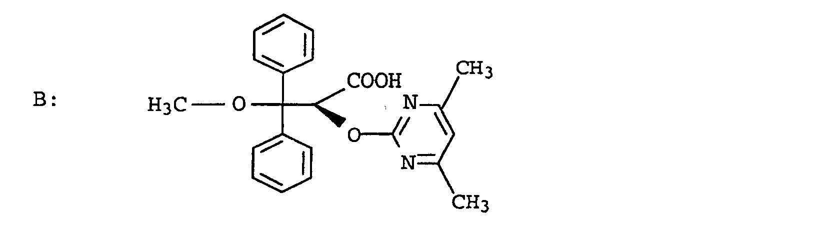

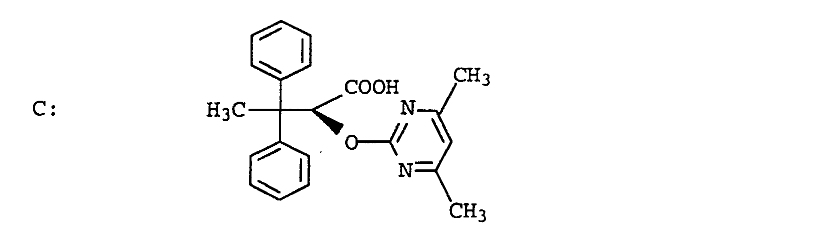

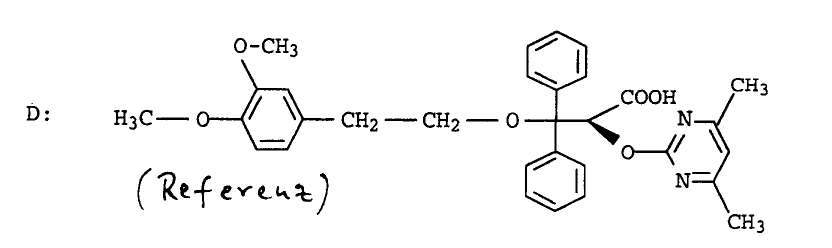

Als Hemmer des Renin-Angiotensin-Systems kommen besonders Angiotensin II-Antagonisten und ganz besonders ACE-Hemmer in Frage.

Für den Zweck der vorliegenden Erfindung geeignete ACE-Hemmer sind Alacepril, Benazepril, Captopril, Cilazapril, Cilazaprilat, Delapril, Enalapril, Enalaprilat, Fosinopril, Lisinopril, Perindopril, Quinapril, Ramipril, Spirapril, Trandolapril und Zofenopril. Bevorzugt unter diesen sind Ramipril und insbesondere Trandolapril.

Für den Zweck der vorliegenden Erfindung geeignete Angiotensin II-Antagonisten sind: Losartan, das Kaliumsalz von 2-Butyl-4-chlor-1-[ [2'-(1H-tetrazol-5-yl) [1,1'-biphenyl]-4-yl]methyl-1H-imidazol-5-methanol; Valsartan, N-(1-Oxopentyl)-N-[[2'-(1H-tetrazol-5-yl)[1,1'-biphenyl-4-yl]methyl-L-valin; Candesartan, 2-Ethoxy-1-[[2'-(1H-tetrazol-5-yl)[1,1'-biphenyl] - 4-yl]methyl]-1H-benzimidazol-7-carbonsäure; 4'-[(2-n-Butyl-6-cyclohexylaminocarbonylamino-benzimidazol-1-yl)methyl]biphenyl-2-carbonsäure; 2-n-Butyl-1-[4-(6-carboxy-2,5-dichlorbenzoylamino)benzyl]-6-N-(methylaminocarbonyl)-n-pentylaminobenzimidazol; 4'-[(1,4'-Dimethyl-2'-propyl-[2,6'-bi-1H-benzimidazol]1'-yl)methyl]-[1,1'-biphenyl]-2-carbonsäure; 4-(Pentafluorethyl)-2-propyl-1-[[2'-(1H-tetrazol-5-yl)[1,1'-biphenyl] -4-yl]methyl]-1H-imidazol-5-carbonsäure ; 4'-[[2-Butyl-4-chlor-5-(hydroxymethyl)-1H-imidazol-1-yl]methyl]-[1,1'-biphenyl]-2-carbonsäure; 2-Ethyl-5,6,7,8-tetrahydro-4-([2'-(-H-tetrazol-5-yl)biphenyl-4-yl]methoxy)chinolin; 2-Ethyl-4-[(2'-(1H-tetrazol-5-yl)biphenyl-4-yl)methoxy]chinolin-hydrochlorid; 1-[[3-Brom-2-(2-(1H-tetrazol-5-yl)phenyl)-5-benzofuranyl]methyl]-2-butyl-4-chlor-1H-imidazol-5-carbonsäure; 1-[[3-Brom-2-[2-[(trifluormethyl)sulphonyl] amino]phenyl-5-benzofuranyl]methyl]-4-cyclopropyl-2-ethyl-1 H-imidazol-5-carboxamid; 5,7-Dimethyl-2-ethyl-3-(2'-(1H-tetrazol-5-yl)(1,1'-biphenyl)-4-yl)-methyl)-3H-imidazo-(4,5-b)pyridin; 5-[4'-(3,5-Dibutyl-1,2,4-triazol-1-ylmethyl)biphenyl-2-yl]-1H-tetrazol; 1,4-Dibutyl-3-[2'-(1H-tetrazol-5-yl)biphenyl-4-ylmethyl]-2,3-dihydro-1H-imidazol-2-on; 2-n-Butyl-4-spirocyclopentan-1-[(2'-(1H-tetrazol-5-yl)biphenyl-4-yl)methyl]-2-imidazolin-5-on; (E)-α-[(2-Butyl-1-[(4-carboxyphenyl)methyl]-1H-imidazol-5-yl]-methylen]-2-thiophenpropansäure; 2-Ethoxy-1-[[2'-(1Htetrazol-5-yl)[1,1'-biphenyl]-4-yl]methyl]-1H-benzimidazol-7-carbonsäure-1-[[(cyclohexyloxy)carbonyl]oxy]ethylester; 2-Propyl-4-[(3-trifluoracetyl)pyrrol-1-yl]-1-[[2'-(2H-tetrazol-5-yl)[1,1'-biphenyl]-4-yl]methyl]-1H-imidazol-5-carbonsäure; und das Kaliumsalz von 2,7-Diethyl-5-[[2'-(1H-tetrazol-5-yl)[1,1'-biphenyl]-4-yl]methyl]-5H-pyrazolo[1,5-b][1,2,4]-triazol.

Als Erkrankungen, die mit einer Vasokonstriktion oder anderen biologischen Wirkungen von Endothelin und/oder Angiotensin II assoziiert sind, seien besonders die Bekämpfung bzw. Verhütung von Koronarerkrankungen, Herz-Kreislauf-Erkrankungen, wie Hypertonie, Herzinsuffizienz, Ischämie (im Herz, Gehirn, Magen-Darm-Trakt, Leber und/oder Niere) oder Vasospasmen genannt. Weitere Beispiele für behandelbare Erkrankungen sind Niereninsuffizienz und Nierenversagen, Dialyse, subarachnoidale Hämorrhagie, Raynaud-Syndrom, portaler Hochdruck und pulmonarer Hochdruck sowie die Behandlung von Magen- und Duodenalulcera und von ulcus cruris, bei denen eine Vasokonstriktion beteiligt ist. Weiter können sie bei Atherosklerose und zur Verhinderung der Restenosierung nach Balloninduzierter Gefäßdilatation eingesetzt werden. Schließlich ist bei Patienten mit Asthma die Konzentration von Endothelin im Bronchialsekret erhöht. Auch bei Migräneanfällen findet man erhöhte Endothelinspiegel im Blutplasma. Die Kombination kann daher auch in diesen beiden Fällen eingesetzt werden.

Bei der Verabreichung der erfindungsgemässen Kombination tritt im Vergleich zu den Einzelsubstanzen eine beachtliche Verstärkung der blutdrucksenkenden Eigenschaften und der Wirkungsdauer auf, die überadditiv ist. Die Dosen der einzelnen Wirkstoffe können so beachtlich reduziert werden. Dadurch ist mit weniger Nebenwirkungen bei der Applikation zu rechnen.

Das Gewichtsverhältnis vom Hemmer des Renin-Angiotensin-Systems zum Endothelin-Antagonisten liegt normalerweise im Bereich von 50:1 bis 1:500, vorzugsweise 10:1 bis 1:100 und insbesonders 2:1 bis 1:50.

Die erfindungsgemässen Kombinationen werden im allgemeinen oral, z.B. in Form von Tabletten, Lacktabletten, Dragees, Hart- und Weichgelatinekapseln, Lösungen, Emulsionen oder Suspensionen verabreicht. Die Verabreichung kann aber auch rektal, z.B. in Form von Suppositorien, oder parenteral, z.B. in Form von Injektionslösungen, erfolgen. Die Verabreichung der Wirkstoffe kann in Form von Präparaten erfolgen, die beide Wirkstoffe zusammen, wie Tabletten oder Kapseln enthalten, oder getrennt als ad-hoc-Kombination von Einzelsubstanzen, die gleichzeitig oder zeitlich abgestuft appliziert werden können.

Zur Herstellung von Tabletten, Lacktabletten, Dragees und Hartgelatinekapseln kann eine erfindungsgemäße Kombination mit pharmazeutisch inerten, anorganischen oder organischen Excipientien verarbeitet werden. Als solche Excipientien kann man für Tabletten, Dragees und Hartgelatinekapseln Lactose, Maisstärke oder Derivate davon, Talk, Stearinsäure oder deren Salze verwenden.

Für Weichgelatinekapseln eignen sich als Excipientien pflanzliche Öle, Wachse, Fette, halbfeste und flüssige Polyole.

Zur Herstellung von Lösungen und Sirupen eignen sich als Excipientien z.B. Wasser, Polyole, Saccharose, Invertzucker, Glukose und dergleichen. Für Injektionslösungen eignen sich als Excipientien Wasser, Alkohole, Polyole, Glyzerin, vegetabile Öle. Für Suppositorien eignen sich als Excipientien natürliche oder gehärtete Öle, Wachse, Fette, halbflüssige oder flüssige Polyole und dergleichen.

Die pharmazeutischen Zubereitungen können daneben noch Konservierungsmittel, Lösevermittler, Stabilisierungsmittel. Netzmittel, Emulgiermittel, Süssmittel, Färbemittel, Aromatisierungsmittel. Salze zur Veränderung des osmotischen Druckes, Puffer, Überzugsmittel und/oder Antioxidantien enthalten.

Die folgenden Versuche zeigen die unerwartet vorteilhaften Eigenschaften der erfindungsgemäßen Kombinationen:

Chronisch instrumentierten männlichen Beagle-Hunden (ca. 14 kg) wurde die Testsubstanz oral als Kapsel im Cross-over design appliziert. Die Kapsel enthielt entweder nichts (Kontrolle n=10), Trandolapril (2 mg/kg; N=10), Verbindung A (10 mg/kg, N=10) oder die Kombinationen aus Trandolapril + Verbindung A (2 + 10 mg/kg; N=10; bzw. 2+0,5 mg/kg; N=5). Zwischen den einzelnen Applikationen wurde eine Auswaschphase von mindestens einer Woche eingehalten. Der Blutdruckverlauf wurde über 6 h registriert. Der systolische (SAP) und diastolische (DAP) Blutdruck in der A. abdominalis [mmHg] wurden gemessen, woraus der mittlere arterielle Blutdruck (MAP) ermittelt wurde. Die Herzfrequenz [min⁻¹] wurde aus dem systolischen Peak des Blutdrucksignals berechnet.

Die Tabelle 1 zeigt, daß in der Kontrollgruppe und in der mit Trandolapril behandelten Gruppe der Blutdruck nicht sinkt. Unter Verbindung A ist eine leichte Blutdrucksenkung zu beobachten, die zu einigen Zeitpunkten signifikant wird. Die Kombination Trandolapril mit dem ET-Antagonisten Verbindung A (2+10 mg/kg) zeigt eine deutliche Blutdrucksenkung, die auch noch in der 2. Kombinationsgruppe mit dem geringeren Anteil an ET_{A}-Antagonistem (nur 0,5 mg/kg anstelle von 2 mg/kg) ausgeprägt ist.

**Tabelle 1:**

| Entwicklung des mittleren arteriellen Blutdrucks (MAP, mmHg) in normotensiven wachen Hunden nach Applikation verschiedener Substanzen, dargestellt sind Mittelwerte | | | | | | | | |
|---|---|---|---|---|---|---|---|---|
| | N | Ausgangswert | 1 h | 2 h | 3 h | 4 h | 5 h | 6 h |
| Placebo | 10 | 103 | 3 | 2 | 2 | 1 | 1 | 1 |
| Verbindung A 10 mg/kg | 10 | 99,6 | -5,9 | -8,9 | -9 | -9,1 | -8,4 | -8,2 |
| Trandolapril 2 mg/kg | 10 | 99,8 | -4,1 | -2,9 | 0,9 | 1,7 | -0,1 | 0,2 |
| Kombination A + T 2+10 mg/kg | 10 | 96 | -18,7 | -30,9 | -30,8 | -30,3 | -28,6 | -23,6 |
| Kombination A + T 0,5 +2 mg/kg | 5 | 97,1 | -3,8 | -12,7 | -14,7 | -11,5 | -10,4 | -4,6 |

In der Kontrollgruppe ändert sich die Herzfrequenz über den Beobachtungszeitraum nicht (Tabelle 2). Unter Verbindung A steigt die Frequenz um etwa 11 Schläge an, was als reflektorische Antwort auf die Blutdruckdrucksenkung interpretiert werden kann. Trandolapril führt ebenfalls trotz der fehlenden Blutdruckwirkung zu einem Frequenzanstieg, der in den Kombinationsgruppen noch stärker ausgeprägt ist.

**Tabelle 2:**

| Entwicklung der Herzfrequenz (min⁻¹) in normotensiven wachen Hunden nach Applikation verschiedener Substanzen, dargestellt sind Mittelwerte | | | | | | | | |
|---|---|---|---|---|---|---|---|---|
| | N | Ausgangswert | 1 h | 2 h | 3 h | 4 h | 5 h | 6 h |
| Kontrolle | 10 | 71 | -3,3 | 2,3 | -1,7 | -4,5 | -2,5 | -5,9 |
| Verbindung A 10 mg/kg | 10 | 73,8 | 3,7 | 11,1 | 10,2 | 8 | 8,3 | 3,1 |
| Trandolapril 2 mg/kg | 10 | 69 | 6,7 | 15,5 | 13 | 10 | 10,3 | 7,5 |
| Kombination A + T 2+10 mg/kg | 10 | 70,6 | 15,6 | 25,4 | 27,2 | 24,1 | 21,6 | 10,5 |
| Kombination A + T 0,5 +2 mg/kg | 5 | 60,3 | 11,1 | 18,8 | 15,1 | 13,2 | 11,1 | 8,3 |

Die nachfolgenden Beispiele erläutern die Erfindung.

### Beispiel 1

Es wurden Lacktabletten folgender Zusammensetzung hergestellt:

| | |
|---|---|
| Verbindung A | 200,0 mg |
| Trandolapril | 2,0 mg |
| Lactose wasserfrei | 30,0 mg |
| Mikrokristalline Cellulose | 30,0 mg |
| Polyvinylpyrrolidon | 20,0 mg |
| Magnesiumstearat | 5,0 mg |
| Polyethylenglykol 6000 | 0,8 mg |
| Eisenoxid gelb | 1,2 mg |
| Titandioxid | 0,3 mg |
| Talk | 0,7 mg |

Verbindung A, Trandolapril, die Lactose, die Cellulose und das Polyvinylpyrrolidon werden feuchtgranuliert und getrocknet. Das gesiebte Granulat wird mit dem Magnesiumstearat gemischt, und die preßfertige Mischung zu ovalen Tablettenkernen zu je 290,0 mg verpreßt. Anschließend werden die Kerne mit Hilfe eines Lackierverfahrens überzogen, bis die Lacktabletten ein Endgewicht von 300 mg erreicht haben.

### Beispiel 2

Herstellung von Hartgelatinekapseln folgender Zusammensetzung:

| | |
|---|---|
| Verbindung A | 250,0 mg |
| Ramipril | 2,5 mg |
| Lactose krist. | 18,0 mg |
| Polyvinylpyrrolidon | 15,0 mg |
| Microkristalline Cellulose | 17,5 mg |
| Natrium-carboxymethylstärke | 10,0 mg |
| Talk | 9,0 mg |
| Magnesiumstearat | 3,0 mg |

Die ersten fünf Bestandteile werden feuchtgranuliert und getrocknet. Das Granulat wird mit der Natrium-carboxymethylstärke, dem Talk und dem Magnesiumstearat gemischt und die Mischung in Hartgelatinekapseln der Größe 1 abgefüllt.

## Patentansprüche

1. Kombination aus einem Endothelin-Antagonisten der Formel (I) 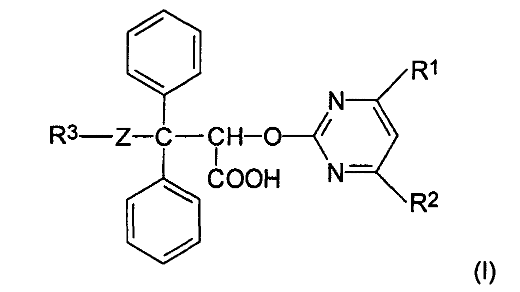 in der die Substituenten folgende Bedeutung haben:
R¹ C₁-C₄-Alkyl, C₁-C₄-Alkoxy;
R² C₁-C₄-Alkyl, C₁-C₄-Alkoxy;
R³ C₁-C₈-Alkyl;
Z Sauerstoff oder eine Einfachbindung;
und einem RAS-Hemmer.

2. Pharmazeutische Zubereitung, enthaltend eine Kombination gemäß Anspruch 1.

3. Herstellung einer pharmazeutischen Zubereitung, **dadurch gekennzeichnet, dass** man ein Gemisch aus einem RAS-Hemmer und einem Endothelin-Antagonisten gemäß Anspruch 1 in eine galenische Darreichungsform bringt.

4. Verwendung einer Kombination von einem RAS-Hemmer und einem Endothelin-Antagonisten gemäß Anspruch 1 zur Herstellung eines Medikaments zur Behandlung von Erkrankungen, die mit einer Vasokonstriktion oder anderen biologischen Wirkungen von Endothelin und/oder Angiotensin II assoziiert sind.

## Claims

1. Combination of an endothelin antagonist of formula (I) 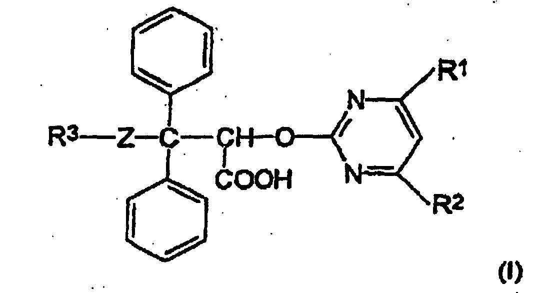 in which the substituents have the following meanings:
R¹ C₁-C₄-alkyl, C₁-C₄-alkoxy;
R² C₁-C₄-alkyl, C₁-C₄-alkoxy;
R³ C₁-C₈-alkyl;
Z oxygen or a single bond;
and an RAS inhibitor.

2. Pharmaceutical preparation comprising a combination according to claim 1.

3. Production of a pharmaceutical preparation,
**characterised in that** a mixture of an RAS inhibitor and an endothelin antagonist according to claim 1 is converted into a galenical dosage form.

4. Use of a combination of an RAS inhibitor and an endothelin antagonist according to claim 1 in the production of a medicament for treating disorders associated with vasoconstriction or other biological effects of endothelin and/or angiotensin II.

## Revendications

1. Combinaison d'un antagoniste de l'endothéline de formule (I) 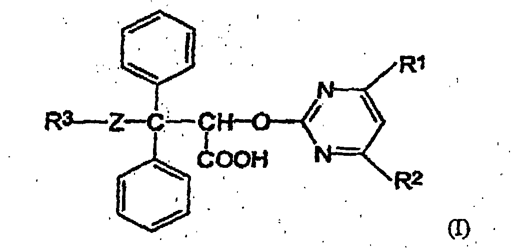 dans laquelle les substituants ont la signification suivante:
R¹ représente un groupe alkyle en C₁-C₄ ou alcoxy en C₁-C₄;
R² représente un groupe alkyle en C₁-C₄ ou alcoxy en C₁-C₄;
R³ représente un groupe alkyle en C₁-C₈;
Z représente un atome d'oxygène ou une liaison simple;
et d'un inhibiteur du SRA (système rénine-angiotensine).

2. Composition pharmaceutique contenant une combinaison selon la revendication 1.

3. Préparation d'une composition pharmaceutique, **caractérisée en ce que** l'on met un mélange d'un inhibiteur du SRA et d'un antagoniste de l'endothéline selon la revendication 1 sous une forme d'administration galénique.

4. Utilisation d'une combinaison d'un inhibiteur du SRA et d'un antagoniste de l'endothéline selon la revendication 1 pour la préparation d'un médicament destiné au traitement de maladies associées à une vasoconstriction ou à d'autres effets biologiques de l'endothéline et/ou de l'angiotensine II.
